# EUROPEAN PATENT APPLICATION

(11) **EP 1 855 111 A1**
(43) Date of publication of application: **14.11.2007**
(21) Application number: 06714611.8
(22) Date of filing: 24.02.2006
(51) Int. Cl.: G01N 33/53, C12N 9/99, C12Q 1/02, C12Q 1/44, G01N 33/15, G01N 33/50

(54) **NOVEL PD MARKER FOR HISTONE DEACETYLASE INHIBITOR**

(30) Priority: 02.03.2005 JP 2005057547
(71) Applicant: Astellas Pharma Inc., Tokyo 103-8411 (JP)
(72) Inventor: MATSUOKA, Hideaki, 2-chome, Chuo-ku Tokyo, 1038411 (JP); ISHII, Yoshinori, 2-chome, Chuo-ku Tokyo, 1038411 (JP); MATSUDA, Kaori, 2-chome, Chuo-ku Tokyo, 1038411 (JP); ARAMORI, Ichiro, 2-chome, Chuo-ku Tokyo, 1038411 (JP); MORI, Hiroaki, 2-chome, Chuo-ku Tokyo, 1038411 (JP)
(74) Representative: Gille Hrabal Struck Neidlein Prop Roos
(86) International application number: PCT/JP2006/303472
(87) International publication number: WO 2006/093053

(57) **Abstract**

It is intended to provide a method for evaluating a pharmacological effect of a histone deacetylase (HDAC) inhibitor, comprising the steps of: (a) preparing a cell expressing CPSF5 protein; (b) contacting the cell with an HDAC inhibitor; (c) measuring the acetylation level of CPSF5 protein in the cell; and (d) comparing the acetylation level measured in the step (c) with the measured acetylation level of CPSF5 protein in a control cell that has not been contacted with the HDAC inhibitor.

## Description

### Technical Field

The present invention relates to a novel PD marker for a histone deacetylase inhibitor.

### Background Art

Major immunosuppressive agents, cyclosporin A (CsA) and tacrolimus (FK506), which are widely used at present in the clinical field for the purpose of suppressing acute rejection after organ transplantation, inhibit the activity of calcineurin, a Ca²⁺/calmodulin-dependent protein phosphatase, through binding to respectively specific immunophilins (for example, cyclophilin for CsA and FKBP12 for FK506). It is known that the nuclear import of NF-AT (a nuclear factor of activated T-cell) is consequently inhibited by inhibition of dephosphorylation of NF-AT, resulting in suppression of the transcriptional activity of IL-2 gene. From the study of such an action mechanism, it has become apparent that the specific suppression of the expression of IL-2 gene at the transcriptional level in an activated T-cell results in suppressesing the organ graft rejection, and this is very important from the viewpoint that a therapeutic effect on various autoimmune diseases and the like is exerted.

In this connection, it has been known that a histone deacetylase (hereinafter referred to as HDAC) that catalyzes deacetylation of histone acts competitively with a histone acetylase in a cellular nucleus and regulates the expression level of various genes through alteration of the chromatin structure. As the results of energetic screening, a large number of compounds exerting an HDAC inhibitory activity have been obtained so far, and they include compounds exerting a prominent inhibitory activity of the production of IL-2 (see Non-patent document 1) which have attracted attention as a candidate for an immunosuppressive agent to complement cyclosporin or tacrolimus. In fact, among the thus screened compounds, a compound exerting an excellent in vivo immunosuppressive action has been found. For example, as disclosed in WO 00/08048 (Patent document 1), FR225497 has been found to exert an excellent effect as a therapeutic agent or a preventive agent for organ graft rejection or an autoimmune disease by its potent immunosuppressive action. In addition to this, it has been suggested to have usefulness as a therapeutic agent or a preventive agent for a large number of other diseases which are considered to be caused by gene expression abnormality. In such diseases, for example, inflammatory diseases, diabetes, diabetic complications, homozygous thalassemia, fibrosis, hepatic cirrhosis, acute promyelocytic leukemia, protozoal infections, cancer and the like are included. In this way, at present, an HDAC inhibitor is considered to be useful as a therapeutic agent or a preventive agent for many diseases, and is one of the medicinal agents which have been energetically studied in the clinical stages.

However, the current evaluation of the effective dose of an HDAC inhibitor in the clinical stages is based only on the putative effective dose on the basis of PK (pharmacokinetics) such as in vitro activity or protein binding ratio, and the establishment of a system for predicting a pharmacological effect of an HDAC inhibitor on the basis of PD (pharmacodynamics), in which reflects the relationship between the effective dose (blood level) and the pharmacological effect, has been demanded.

Patent document 1: International Publication No. WO 00/08048
Non-patent document 1: I. Takahashi et al., The Journal of Antibiotics 49, 453-457 (1995)

### Disclosure of the invention

### Problems to be solved by the Invention

In view of the above circumstances, the present inventors have identified a novel PD marker protein for an HDAC inhibitor for establishing a system for predicting a pharmacological effect of an HDAC inhibitor on the PD basis. This protein is a protein called CPSF5 involved in mRNA processing, and a novel protein as a substrate for HDAC. CPSF5 can be easily prepared compared with histone which is a typical substrate for HDAC. Further, CPSF5 sufficiently satisfies the requirements as a PD marker, for example, the acetylation level of CPSF5 biologically correlates with a pharmacological effect of an HDAC inhibitor, sensitive and simple detection can be achieved with the use of a sample which can be easily obtained clinically (such as PBMC or a biopsy), quantitative detection can be achieved in an immunoassay, a variability between animal species is not observed, and so on. In the prediction of a pharmacological effect of an HDAC inhibitor on the basis of PK/PD using such a PD marker, it becomes possible to accurately predict a clinical effect in the early stage of a clinical study and to establish an optimal dose for individual humans in the clinical field, and further, it is considered that a pharmacological effect of an HDAC inhibitor can be maximized with a minimum risk.

### Means for Solving the Problems

One aspect of the present invention is to provide a method for evaluating a pharmacological effect of a histone deacetylase (HDAC) inhibitor, comprising the steps of:
(a) preparing a cell expressing CPSFS protein;
(b) contacting the cell with an HDAC inhibitor; and
(c) measuring the acetylation level of CPSF5 protein in the cell.

In one embodiment, the cell is a peripheral blood mononuclear cell.

In one embodiment, the peripheral blood mononuclear cell is a human peripheral blood mononuclear cell.

In one embodiment, the pharmacological effect of an HDAC inhibitor is an immunosuppressive effect.

In one embodiment, the acetylation level of CPSF5 protein is measured by an immunoassay using an anti-acetylated lysine antibody.

Another aspect of the present invention is to provide a method for evaluating a pharmacological effect of a histone deacetylase (HDAC) inhibitor, comprising the steps of:
(a) administering an HDAC inhibitor to a mammal;
(b) collecting a cell expressing CPSF5 protein from the mammal; and
(c) measuring the acetylation level of CPSF5 protein in the cell.

In one embodiment, in the step (b), the cell is collected multiple times with time, and in the step (c), the acetylation level of CPSF5 protein in the cell is measured for each cell collected at each time.

In one embodiment, the cell is a peripheral blood mononuclear cell.

In one embodiment, the mammal is a human.

In one embodiment, the mammal is a non-human mammal.

In one embodiment, the pharmacological effect of an HDAC inhibitor is an immunosuppressive effect.

In one embodiment, the acetylation level of CPSF5 protein is measured by an immunoassay using an anti-acetylated lysine antibody.

Another aspect of the present invention is to provide a method for screening a histone deacetylase (HDAC) inhibitor, comprising the steps of:
(a) preparing a cell expressing CPSF5 protein;
(b) contacting the cell with a test substance; and
(c) measuring the acetylation level of CPSF5 protein in the cell.

### Advantage effect of the Invention

By the prediction of a pharmacological effect of an HDAC inhibitor on the basis of PK/PD utilizing the present invention, it becomes possible to accurately predict a clinical effect in the early stage of a clinical study and to establish an optimal dose for individual humans in the clinical field, and further, a pharmacological effect of an HDAC inhibitor can be maximized with a minimum risk.

### Brief Description of the Drawings

[Fig. 1] Fig. 1 shows the detection of a protein (p25) in which the enhancement of acetylation was observed at 4 hours after the addition of an HDAC inhibitor to normal rat PBMC.
[Fig. 2] Fig. 2 shows a method for identifying p25 protein.
[Fig. 3] Fig. 3 shows the identification results for p25 protein.
[Fig. 4] Fig. 4 shows the Mascot search results for p25 protein.
[Fig. 5] Fig. 5 shows an amino acid sequence of CPSF5 and a schematic view of its function.
[Fig. 6] Fig. 6 shows the change with time in the acetylation level of CPSF5 in rat PBMC after the administration of an HDAC inhibitor (correspondence to the plasma level of an HDAC inhibitor).
[Fig. 7] Fig. 7 shows the acetylation of CPSF5 in rat PBMC at 4 hours after the administration of an HDAC inhibitor (in vivo dose dependency).
[Fig. 8] Fig. 8 shows the acetylation of CPSF5 in a clinical material.
[Fig. 9] Fig. 9 shows the correlation between the change in the acetylation level of CPSF5 in a transplantation model and a pharmacological effect of an HDAC inhibitor.

### Best Mode for Carrying Out the Invention

Hereinafter, the present invention will be described in detail.

The term "CPSF5 protein" or "CPSF5" as used herein refers to a nuclear protein involved in mRNA processing named cleavage and polyadenylation specific factor 5, 25 kD subunit. CPSF5 protein has so far been identified in human, rat, mouse and so on, and its amino acid sequence is highly conserved among species. Examples of the CPSF5 protein to be used in the method of the present invention include such a naturally occurring CPSF5 protein or modified proteins thereof (such as allelic variants and polymorphic variants), or variants or mutants produced by artificially deleting, adding, substituting or inserting one or several amino acids or modifying an amino acid by a method such as a genetically engineering technique or chemical modification. As described above, CPSF5 is a nuclear protein involved in mRNA processing, and is expressed in cells (such as peripheral blood mononuclear cells (PBMC), splenocytes, vascular smooth muscle cells, Jurkat cells, THP-1 cells, HEL cells and HEK293 cells) derived from blood and almost all the tissues (for example, tissues such as blood vessels, heart, brain, lung, liver, kidney, small intestine, large intestine, urinary bladder, testis, ovary, spleen and thymus). The "cell expressing CPSF5 protein" that can be used in the method of the present invention can be easily selected from any various body fluids or tissue biopsies by using an expression analysis method known to a person skilled in the art such as a Southern blot analysis or a Northern blot analysis with the use of a polynucleotide encoding known CPSF5, or a Western blot analysis with the use of an antibody against CPSF5 protein. Preferably, from the viewpoint of the simplicity and accuracy, the "cell expressing CPSF5 protein" to be used in the method of the present invention is a peripheral blood mononuclear cell (PBMC). Alternatively, by recombinantly expressing CPSFS gene in any cell, a cell expressing CPSF5 protein is produced, and the resulting cell may be used in the present invention.

As the "HDAC inhibitor" to be used in the method of the present invention, any HDAC inhibitors known in this field can be exemplified, and examples thereof include those described in WO 01/38322, WO 01/070675, WO 00/071703, WO 00/08048, WO 03/015819, WO 03/057722, WO 04/063169 and the like. HDAC is an enzyme that converts acetylated lysine into lysine in histon by hydrolyzing the acetyl group in the acetylated lysine residue of the histone, and is an important protein involved in the regulation of gene transcription through alteration of the chromatin structure. It has been suggested that such an HDAC inhibitor that inhibits the activity of HDAC has an effect as a therapeutic agent or a preventive agent for many diseases considered to be caused by gene expression abnormality. Examples of such a disease include inflammatory diseases, diabetes, diabetic complications, homozygous thalassemia, fibrosis, hepatic cirrhosis, acute promyelocytic leukemia, protozoal infections, cancer and the like. Further, it has been confirmed that the HDAC inhibitor exerts an immunosuppressive effect by inhibiting the expression of IL-2 gene at the transcriptional level in an activated T-cell, and it is useful also for treating or preventing rejection upon transplantation of an organ or a tissue such as heart, kidney, liver, bone marrow, skin, cornea, lung, pancreas, small intestine, limb, muscle, nerve, intervertebral disc, trachea, myoblast or cartilage, graft-versus-host reaction caused by a bone-marrow transplantation, an autoimmune disease or the like. The method of the present invention using acetylation of CPSFS protein as an indicator can be used for evaluation of a pharmacological effect of an HDAC inhibitor against the above-mentioned various diseases. In one embodiment, the pharmacological effect of an HDAC inhibitor that can be evaluated by using the method of the present invention is an immunosuppressive effect on organ graft rejection, an autoimmune disease or the like.

The deacetylation reaction of CPSF5 protein by HDAC is a reaction of deacetylating a lysine residue in CPSF5, and the acetylation level of CPSFS protein can be measured by any of various methods known to a person skilled in the art. For example, as described in the following Examples, the acetylation level of CPSF5 protein can be quantitatively measured by an immunoassay such as a Western blot assay using an anti-acetylated lysine antibody. Alternatively, such an acetylation reaction can also be quantitatively measured by an assay method using an acetyl group labeled with a radioisotope such as ³H or ¹⁴C or an assay method utilizing the EIA/ELISA method. In the former method, for example, sodium acetate or the like (an acetyl group donor), which is labeled with ³H or ¹⁴C, is incorporated into a cell, and an acetylated protein in the cell is labeled with ³H or ¹⁴C. Thereafter, CPSF5 protein is purified by utilizing an immunoprecipitation method and so on, and the acetylation of CPSFS can be detected. In the latter method (for example, a sandwich ELISA method using an anti-CPSF5 antibody and an anti-acetylated lysine antibody), for example, a sample is added to a 96-well plate in which an anti-CPSF5 antibody has been immobilized, and a reaction is allowed to proceed. Thereafter, the plate is washed, and an anti-acetylated lysine antibody is added to the plate, and a reaction is allowed to proceed. Then, the plate is washed, and a color is allowed to develop, whereby the acetylation of CPSF5 can be detected.

In another aspect of the invention, an HDAC inhibitor is administered to a mammal and a cell expressing CPSF5 protein is collected from the mammal after the administration thereof, and then, the acetylation level of this CPSF5 protein may be measured. As the "mammal" to be used in the method of the present invention, a human, a monkey, a dog, a rat, a mouse or another appropriate mammal can be used, and the administration of an HDAC inhibitor to a mammal can be carried out orally or parenterally by any administration method known in the art. For example, in a pre-clinical study or the early stage of a clinical study, by using a non-human mammal in the method of the present invention, a pharmacological effect of an HDAC inhibitor on a human may be predicted. Alternatively, by administering an HDAC inhibitor to a human in the method of the present invention, it is also possible to determine an optimal dose of the HDAC inhibitor to the individual humans. In a preferred embodiment, after an HDAC inhibitor is administered to a mammal, a cell expressing CPSF5 protein is collected multiple times with time, and the acetylation level of CPSFS protein in the cell is measured for each cell collected at each time.

In the method of the present invention, a control sample or a control mammal which is of the same type as a biological sample or a mammal contacted with or administered with an HDAC inhibitor, and is not contacted with or administered with the HDAC inhibitor, may be used as a comparative control for the acetylation level of CPSF5. It is evaluated that the higher the acetylation level of CPSF derived from a test sample compared with the acetylation level of CPSF derived from a control sample is, the higher the pharmacological effect of an HDAC inhibitor is.

Alternatively, by using the method of the present invention, it is also possible to screen an HDAC inhibitor. As a test substance in the screening method, other than a known or novel synthetic compound, peptide, protein or the like, for example, a cell extract, a cell culture supernatant or the like can be used.

Hereinafter, Examples will be described for explaining the present invention in more detail, however, the invention is not limited to these Examples.

### Examples

### Example 1: Detection of a protein in which the enhancement of acetylation is observed in vitro by a treatment with an HDAC inhibitor in rat PBMC

In order to establish a PD assay system that enables the elucidation of the relationship between a pharmacological effect of an HDAC inhibitor and a dose thereof, screening for a novel PD marker for an HDAC inhibitor was carried out. The PD marker has to be able to be detected simply and sensitively using a sample which can be clinically obtained. Therefore, it was decided to examine a change in the acetylation of an intracellular protein in a peripheral blood mononuclear cell (PBMC) by a treatment with an HDAC inhibitor.

The peripheral blood was collected from a Lewis rat, and PBMC was isolated using Ficoll-Paque PLUS (Amersham Biosciences). The concentration of the isolated PBMC was adjusted to 1 x 10⁶ cells/mL with 10% FCS-RPMI 1640 medium (SIGMA) and inoculated into a 6-well plate (IWAKI). The cells were cultured under the conditions of 37°C, 5% CO₂ and saturated humidity in the presence of DMSO (control), Compound X (10, 30, 100 or 300 nM), which is an HDAC inhibitor having an immunosuppressive activity, and Compound Y (100 nM), which is an immunosuppressive agent with no HDAC inhibitory activity for 4 hours, and then, sampling was carried out. Each sample was solubilized in a RIPA buffer (50 mM Tris-HCl (pH 8.0), 150 mM NaCl, 1.0% NP-40, 0.5% deoxycholeate, 0.1% SDS and a protease inhibitor), and a total cell lysate was prepared. After the obtained total cell lysate was subjected to SDS-PAGE (using NuPAGE 4 - 12%, Bis-Tris Gel, MES buffer (Invitrogen)), it was transferred to a PVDF membrane (Immobilon-P (Millipore)) by a semi-dry method (using TRANS-BLOT SD (BIO-RAD)). Then, Western blotting using an anti-acetylated lysine antibody (Cell signaling, using a 1000-fold dilution) was carried out. In order to confirm that the amount of the electrophoresed protein in each lane is equal, Western blotting using an anti-actin antibody (Chemicon, using a 1000-fold dilution) was also carried out.

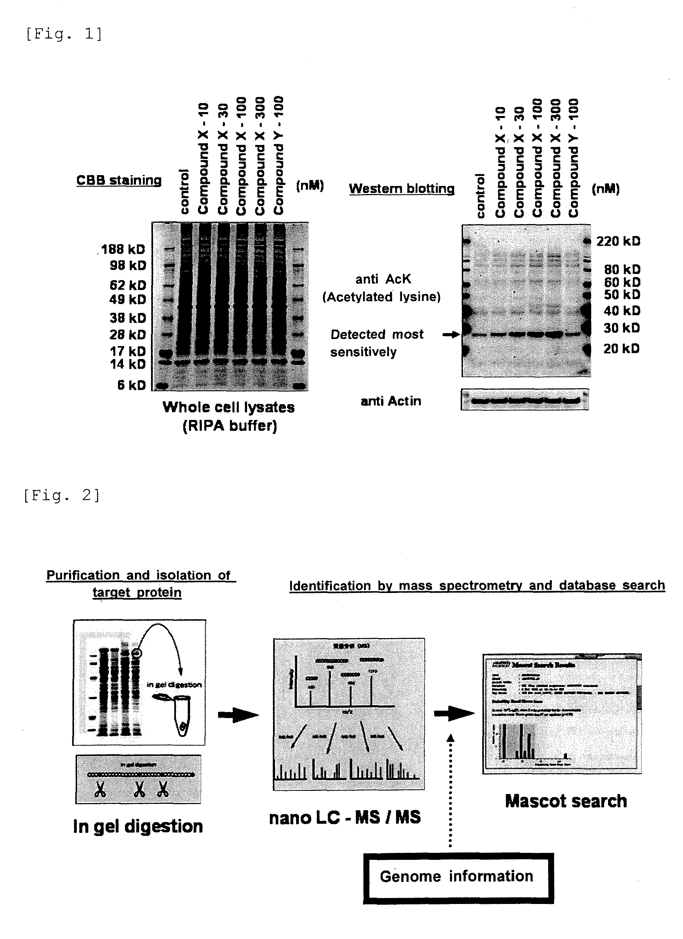

Several proteins in which the dose-dependent enhancement of acetylation by a treatment with an HDAC inhibitor (Compound X) were observed (Fig. 1). Among them, particularly the acetylation of a protein of about 25 kD (p25) was detected at the highest sensitivity, and it was decided to further analyzing it as a novel PD marker candidate for an HDAC inhibitor. Incidentally, the acetylation of p25 was not changed at all by a treatment with an immunosuppressive agent which does not have an HDAC inhibitory activity (Compound Y).

### Example 2: Identification of acetylated 25 kD protein

Identification of the 25 kD protein (p25) which was found as a novel PD marker candidate for an HDAC inhibitor in rat PBMC was attempted. The identification of the protein was carried out by techniques of purification and isolation of the target protein, mass spectrometry and database search with the use of nano LC-MS/MS and Masot search (Fig. 2). Further, because the amount of database information of humans is larger than that of rats, a sample derived from a human was used.

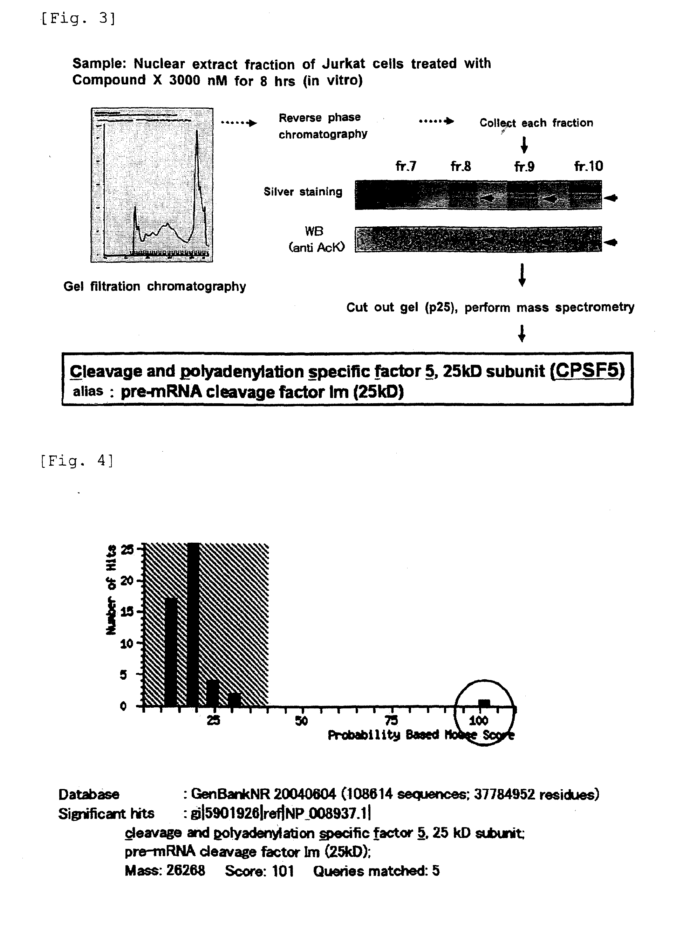

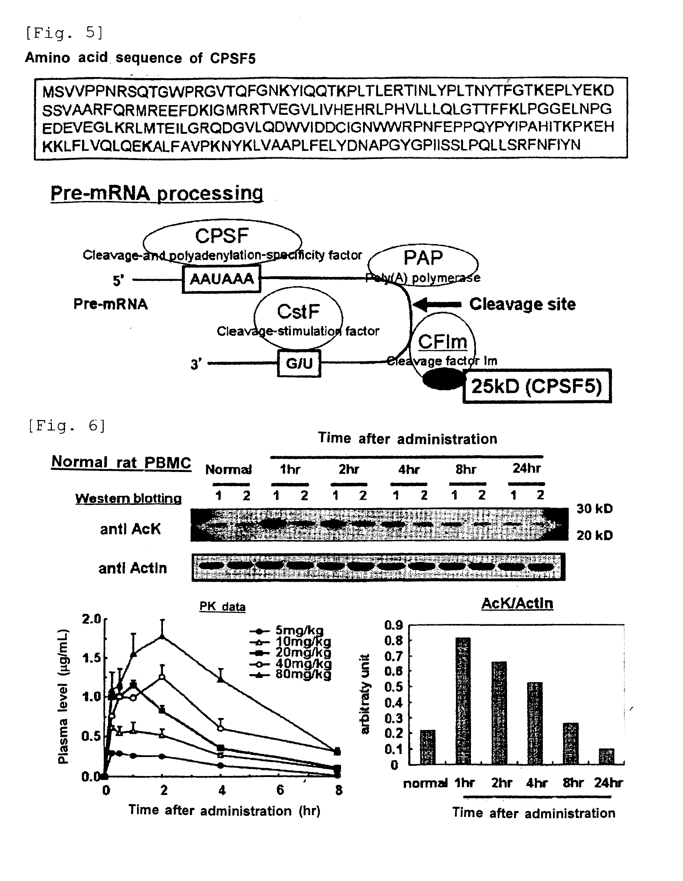

A human T-cell line, Jurkat cell (ATCC: TIB-152) was cultured under the conditions of 37°C, 5% CO₂ and saturated humidity in the presence of 3000 nM Compound X for 8 hours, and then, sampling was carried out. A cytoplasmic fraction (dissolved in 25 mM Tris-HCl (pH 8.0), 150 mM NaCl and 0.1% NP-40) and a nuclear fraction (after the cytoplasmic fraction was removed, the residue was dissolved in 25 mM Tris-HC1 (pH 8.0), 150 mM NaCl and 0.1% Triton X-100) were prepared. When Western blotting was carried out using an anti-acetylated lysine antibody, an acetylated protein of about 25 kD (p25) was detected in the nuclear fraction. Accordingly, the nuclear fraction was concentrated using Centricon (YM-10, Millipore) and purified by gel filtration chromatography (AKTA, Superdex 200HR PBS). Further, the resulting matter was purified by reverse phase chromatography (Phenyl-TOYOPEARL), and a fraction in which acetylated p25 can be detected was confirmed by Western blotting using an anti-acetylated lysine antibody (Fig. 3). After the fraction was subjected to SDS-PAGE, silver staining was carried out. Then, a band whose size coincided with that of acetylated p25 detected by Western blotting was cut out, and a sample for MS analysis was prepared. As a result of LC-MS/MS analysis and Mascot search, it was revealed that p25 is CPSFS (cleavage and polyadenylation specific factor 5, 25 kD subunit) (Fig. 4 and Fig. 5).

### Example 3: Change with time in the acetylation of CPSF5 in rat PBMC after the administration of an HDAC inhibitor (correspondence to the drug plasma level)

For the purpose of confirming whether or not the acetylation of CPSF5 in PBMC corresponds to an increase in the plasma level of an HDAC inhibitor in vivo and to examine at what point after the administration thereof the acetylation level of CPSF5 can be detected, the acetylation level of CPSF5 in PBMC after the administration of Compound X at a dose of 20 mg/kg in a rat was measured with time, and comparison thereof with a PK parameter (a change in plasma level) was carried out.

To a Lewis rat, Compound X was administered in a single dose of 20 mg/kg, and the blood was collected at 1, 2, 4, 8 and 24 hours after the administration thereof. Then, PBMC was isolated using Ficoll-Paque PLUS (Amersham Biosciences). As a control group, PBMC of a normal rat was used. The procedure thereafter was carried out in the same manner as in Example 1.

A change in the acetylation level of CPSF5 after administration of Compound X at a dose of 20 mg/kg showed substantially the same behavior as the change in the plasma level (Fig. 6).

From the above results, it was revealed that in order to accurately quantify the change in the acetylation level of CPSF5, it is necessary to measure it before administration and at a time when the plasma level of the drug is high.

### Example 4: Acetylation of CPSF5 in rat PBMC at 4 hours after administration of an HDAC inhibitor (in vivo dose dependency)

As for the acetylation of CPSF5 in PBMC, the in vivo dose dependency of an HDAC inhibitor was examined.

To a Lewis rat, a vehicle (0.5% MC) and Compound X (5, 10, 20 or 40 mg/kg) were administered in a single dose, and at 4 hours thereafter, the blood and the spleen were isolated. As for the blood, PBMC was isolated using Ficoll-Paque PLUS (Amersham Biosciences). As for the spleen, it was homogenized using a glass homogenizer and subject to hemolytic treatment, and then, spleen cells were prepared by carrying out centrifugation. The procedure thereafter was carried out in the same manner as in Example 1.

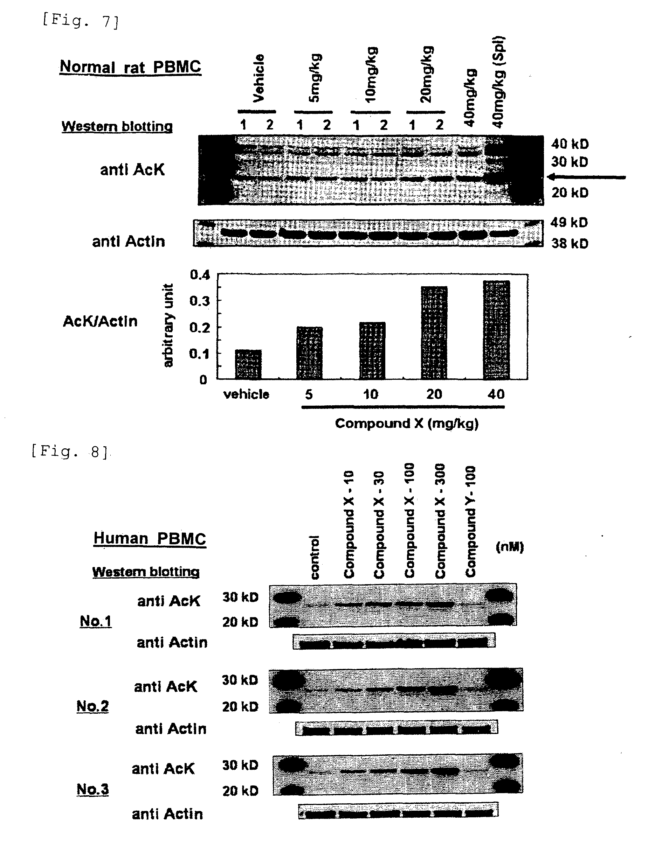

At 4 hours after the single administration of Compound X, the dose-dependent enhancement of acetylation of CPSF5 by Compound X was observed also in vivo in the same manner as in vitro (Fig. 7).

### Example 5: Acetylation of CPSFS in a clinical material

Whether or not the acetylation of CPSF5 in human PBMC which is to be used for the actual evaluation in clinical practice can be detected was examined.

The peripheral blood was collected from three normal subjects (volunteers) in an amount of 30 mL for each subject, and PBMC was isolated using Ficoll-Paque PLUS (Amersham Biosciences). The concentration of the isolated PBMC was adjusted to 1 x 10⁶ cells/mL with 10% FCS-RPMI 1640 medium (SIGMA) and inoculated into a 6-well plate (IWAKI). The cells were cultured under the conditions of 37°C, 5% CO₂ and saturated humidity in the presence of DMSO (control), Compound X (10, 30, 100 or 300 nM) or Compound Y (100 nM) for 4 hours, and then, sampling was carried out. The procedure thereafter was carried out in the same manner as in Example 1.

The dose-dependent enhancement of acetylation of CPSF5 by an HDAC inhibitor (Compound X) was observed also in human PBMC in the same manner as in rat PBMC. Incidentally, the acetylation level of CPSF5 was not changed by a treatment with Compound Y, which is an immunosuppressive agent with no HDAC inhibitory activity (Fig. 8).

From the above results, it was shown that the acetylation of CPSF5 can be utilized as a PD marker for evaluating the action of an HDAC inhibitor (Compound X) also in clinical practice.

### Example 6: Correlation between acetylation of CPSF5 and pharmacological effect

The correlation between the acetylation of CPSF5 in PBMC and a pharmacological effect (efficacy) of an HDAC inhibitor in vivo was examined.

Compound X and Compound Z have a substantially equivalent HDAC inhibitory activity and T-cell proliferation inhibitory activity in vitro, and in terms of the oral absorption in rats, Compound Z is better than Compound X. However, in a rat cardiac transplantation model, Compound X at a dose of 20 mg/kg exerts a remarkable effect on prolonging graft survival as a single agent, however, Compound Z at a dose of 20 mg/kg does not exert an effect on prolonging graft survival.

By using these two compounds, the correlation between the effect of a single agent on prolonging graft survival and the acetylation level of CPSF5 in PBMC in a rat cardiac transplantation model was examined.

To a Lewis rat, Compound X or Compound Z was administered in a single dose of 20 mg/kg, and the blood was collected at 2 and 4 hours after the administration thereof. Then, PBMC was isolated using Ficoll-Paque PLUS (Amersham Biosciences). As a control group, PBMC of a normal rat was used. The procedure thereafter was carried out in the same manner as in Example 1.

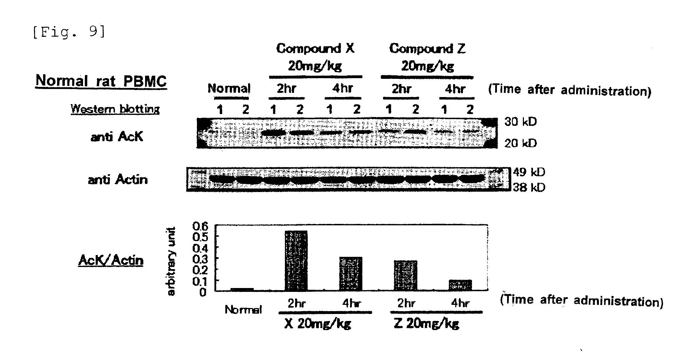

The acetylation level of CPSF5 in PBMC of the group administered with 20 mg/kg of Compound X that exerts effectiveness in a transplantation model was higher at both 2 and 4 hours after the administration thereof compared with that of a group administered with 20 mg/kg of Compound Z that does not exert effectiveness in the same model (Fig. 9).

From the above results, it was shown that the change in the acetylation level of CPSF5 correlates with the pharmacological effect of an HDAC inhibitor, and can be utilized as a PD marker for evaluating the action of an HDAC inhibitor.

### Industrial Applicability

According to the present invention, by the prediction of a pharmacological effect of an HDAC inhibitor on the basis of PK/PD, it becomes possible to accurately predict a clinical effect in the early stage of a clinical study and to establish an optimal dose for individual humans in the clinical field, and further, it is possible to maximize a pharmacological effect of an HDAC inhibitor with a minimum risk.

## Claims

1. A method for evaluating a pharmacological effect of a histone deacetylase (HDAC) inhibitor, comprising the steps of:
(a) preparing a cell expressing CPSF5 protein;
(b) contacting the cell with an HDAC inhibitor; and
(c) measuring the acetylation level of CPSF5 protein in the cell.

2. The method according to claim 1, wherein the cell is a peripheral blood mononuclear cell.

3. The method according to claim 2, wherein the peripheral blood mononuclear cell is a human peripheral blood mononuclear cell.

4. The method according to claim 1, wherein the pharmacological effect of an HDAC inhibitor is an immunosuppressive effect.

5. The method according to claim 1, wherein the acetylation level of CPSF5 protein is measured by an immunoassay using an anti-acetylated lysine antibody.

6. A method for evaluating a pharmacological effect of a histone deacetylase (HDAC) inhibitor, comprising the steps of:
(a) administering an HDAC inhibitor to a mammal;
(b) collecting a cell expressing CPSF5 protein from the mammal; and
(c) measuring the acetylation level of CPSF5 protein in the cell.

7. The method according to claim 6, wherein in the step (b), the cell is collected multiple times with time, and in the step (c), the acetylation level of CPSF5 protein in the cell is measured for each cell collected at each time.

8. The method according to claim 6, wherein the cell is a peripheral blood mononuclear cell.

9. The method according to claim 6, wherein the mammal is a human.

10. The method according to claim 6, wherein the mammal is a non-human mammal.

11. The method according to claim 6, wherein the pharmacological effect of an HDAC inhibitor is an immunosuppressive effect.

12. The method according to claim 6, wherein the acetylation level of CPSF5 protein is measured by an immunoassay using an anti-acetylated lysine antibody.

13. A method for screening a histone deacetylase (HDAC) inhibitor, comprising the steps of:
(a) preparing a cell expressing CPSF5 protein;
(b) contacting the cell with a test substance; and
(c) measuring the acetylation level of CPSF5 protein in the cell.
